# EUROPEAN PATENT APPLICATION

(11) **EP 3 964 143 A1**
(43) Date of publication of application: **09.03.2022**
(21) Application number: 21154590.0
(22) Date of filing: 01.02.2021
(51) Int. Cl.: A61B 17/11

(54) **CLAMPING DEVICE AND APPLICATOR FOR JOINING FLEXIBLE TUBULAR WALLS**

(30) Priority: 02.09.2020 EP 20194149
(71) Applicant: Technische Universität München, 80333 München (DE)
(72) Inventor: Steger, Jana, 80469 München (DE); Ostler, Daniel, 81371 München (DE); Wilhelm, Dirk, 80469 München (DE); Ficht, Stefanie, 80798 München (DE); Eblenkamp, Markus, 85635 Höhenkirchen-Siegertsbrunn (DE); Mela, Petra, 85748 Garching bei München (DE)
(74) Representative: Lucke, Andreas

(57) **Abstract**

A clamping device for joining flexible tubular walls comprises a first clamping element and a second clamping element, wherein the first clamping element comprises a magnetically soft connection portion, and the second clamping element comprises a magnetically hard connection portion, and wherein the first clamping element and the second clamping element are configured to define a common central through-going passage through the first clamping element and the second clamping element, when the magnetically soft connection portion of the first clamping element is coupled to the magnetically hard connection portion of the second clamping element.

## Description

### FIELD OF THE INVENTION

The present invention is in the field of surgical support devices and implants. More precisely, the present invention relates to surgical devices for the transluminal creation of a surgical anastomosis.

### BACKGROUND

One of the most frequent surgical interventions in colorectal surgery is the resection of a pathological bowel section, such as the surgical removal of cancerous tissue. After the pathological bowel section has been removed, the resulting bowel endings need to be surgically reconnected to restore the natural function of the gastrointestinal tract. This connection of the two lumina is referred to as a surgical anastomosis.

Conventionally, the surgical anastomosis between the disconnected bowel sections is established by sutures or stapling techniques, thereby joining the bowel sections to each other at a plurality of joining points.

However, most available techniques to restore a fluid-tight connection between the tissue sections using sutures or staplers require open surgery and are associated with postoperative complications, such as stenosis or leakage, as well as surgical trauma. While the surgical trauma can be reduced through laparoscopic surgery techniques, the required surgical skill for these interventions can be significant. Recent efforts have therefore focused on supporting the surgical procedure by specialized devices, such as specialized staple applicators for transluminal insertion towards the anatomical site of the resection and for reducing the required surgical skill for the (surgical) intervention as well as the risk of surgical errors.

For example, US 6,503,259 B2 discloses a stapling system configured to pierce inverted tissue sections of both the oral and aboral lumen with a circular configuration of pins and receiving elements. The pins and receiving elements are arranged as a plurality of separate and unconnected staple elements in order to allow the anastomosis to remain flexible, while a pre-determined diameter of the surgical anastomosis is defined by a gasket held in place by the staple elements. A dispenser is provided which is inserted into the lumen and drives the pins into the receiving elements in a fixed spatial configuration in order to create a surgical anastomosis.

US 7,338,505 B2 discloses an end-to-end anastomosis stapling device comprising distal and proximal stapler portions, which are expandable to increase the accuracy of the anastomosis while allowing passage through the bowel.

As an alternative, surgical implants can be inserted into the anatomical cavities for compressing and joining aboral and oral tissue sections and for inducing and supporting the healing process.

For example, US 8,623,036 B2 discloses a device for creating an anastomosis using magnets (magnamosis device). The device comprises two ring-shaped clamping elements with permanent magnets mounted therein. The magnets are used to join and compress the disconnected bowel sections via magnetic forces when the tissue is draped over the respective clamping elements. The ring-shaped clamping elements feature mating surfaces having different curvatures in order to induce non-uniform compressive forces onto the tissue sections.

US 10,517,600 B2 discloses a self-assembling magnetic anastomosis device formed by magnetic sections pivotally connected to each other. The pivotally connected sections enable storage and transport of the clamping elements in a folded or semi-linear state towards a target position. When the device is released, the internal spring forces of the joints bias the magnetic sections into a substantially circular shape of a self-assembling polygonal clamping element. Two corresponding clamping elements may then be used to join disconnected tissue sections to create a surgical anastomosis via magnetic forces. Different shapes of the resulting implant can be pre-determined by the size and number of sections in the clamping elements.

### SUMMARY OF THE INVENTION

However, the known surgical implants are still regularly associated with postoperative complications, such as leakage from the surgical anastomosis, and require significant surgical skill during application. In addition, the approach of the implant towards the anatomical site of the intervention and the manipulation of the implant within the body have remained challenging. Most of the prior art devices are also not adapted for transluminal application, i.e. for the application via a natural orifice of the body to minimize surgical trauma, or said application is limited to proximal cavity sections due to the difficulty of force transmission along a winding path and through the lumen to deform metallic staples.

In view of this state-of-the-art, the object of the invention is to provide an improved system for end-to-end anastomosis compatible with transluminal surgery which reduces the risk of postoperative complications while reducing the required surgical skill for the intervention as well as the risk of surgical errors.

This object is solved by a clamping device, an applicator and a system of the clamping device and the applicator according to the independent claims. The dependent claims relate to preferred embodiments.

According to a first aspect, the invention relates to a clamping device for joining flexible tubular walls. The clamping device comprises a first clamping element and a second clamping element. The first clamping element comprises a magnetically soft connection portion, and the second clamping element comprises a magnetically hard connection portion. The first clamping element and the second clamping element are configured to define a common central through-going passage through the first clamping element and the second clamping element, when the magnetically soft connection portion of the first clamping element is coupled to the magnetically hard connection portion of the second clamping element.

The clamping device may be applied as an implant for creating a surgical anastomosis using the magnetic attraction between the first clamping element and the second clamping element to clamp end sections of the flexible tissue walls of anatomical lumina together and to induce merging of the disconnected tubular walls. The clamping elements may form respective implant components, e.g. to lie in oral and aboral tissue sections at the anatomical site of the surgical resection.

By clamping the flexible tubular walls via magnetic forces, the alignment and locking of the clamping elements to each other can be achieved without the application of external mechanical forces, and the tissue sections may be clamped between the clamping elements without piercing the tissue sections. Hence, the clamping device may be deployed also in deep cavities where controlled connection of the clamping elements via mechanical forces transmitted through the lumen would be challenging. In addition, magnetic connection elements may self-align and may thus facilitate application of the clamping device.

As opposed to prior devices, the clamping device clamps the flexible (tissue) walls through magnetic interaction between a magnetically hard connection portion, i.e. a permanently magnetic portion, and a magnetically soft connection portion, such as a polarizable ferromagnetic connection element. The inventors found that the magnetically soft connection portion enables the disconnection of the first clamping element and the second clamping element through locally applied magnetic forces by reversing the magnetization of the magnetically soft connection portion of the first clamping element and inducing a repellent force between the two clamping elements.

Accordingly, the clamping device can be repositioned when the clamping elements have been incorrectly connected, e.g. when the compressive pressure is insufficient or too high, or when the connection between the flexible tubular walls is leaking due to folds of clamped tissue or an incorrect overlap between tubular wall sections, e.g. resulting in unclamped wall segments. As the disconnection of the clamping elements can be substantially achieved with magnetic forces, e.g. generated by a local electromagnet, the clamping device may be disconnected without requiring the transmission of mechanical forces through the flexible tubular walls to overcome the connection forces between the clamping elements. Hence, the clamping elements may be selectively disconnected from each other also when the clamping device is applied transluminally in a deep cavity, where the application of large mechanical forces, e.g. requiring force transmission all along the entire length of the anatomical lumen, such as the bowel, may be limited.

In preferred embodiments, the clamping device is configured as a transluminally applied implant for joining anatomical lumina, and wherein the first clamping element and the second clamping element in particular each comprise a biocompatible coating.

For example, the first clamping element and the second clamping element may be transluminally approached to the anatomical site of resection for creating the surgical anastomosis. The surgical anastomosis can be created with the clamping device by inserting the first clamping element into a first lumen to be joined to a second lumen via the surgical anastomosis, and by inserting the second clamping element into the second lumen. Respective tissue walls of the first and second lumina may then be folded over the first clamping element and the second clamping element, respectively, to align the tissue walls on facing surfaces of the first clamping element and the second clamping element. The relative spatial positions of the clamping elements may then be fixed by connecting the first clamping element and the second clamping element via the respective magnetically soft and magnetically hard connection portions to hold the tissue walls of the first and second lumina against each other. The through-going passage formed by the first clamping element and the second clamping element may enable the restoration of the natural biological function through the anastomosis with the clamping device holding the tissue walls against each other.

The magnetically hard connection portion and the magnetically soft connection portion each may be enclosed in a biocompatible clamping body which may enclose the magnetically hard connection portion and the magnetically soft connection portion as a biocompatible coating. For example, the biocompatible clamping body may be formed of a biocompatible polymer. Additionally or alternatively, the clamping body, the magnetically hard connection portion and/or the magnetically soft connection portion may be covered by a biocompatible coating to enable the deployment of the clamping device as an implant for creating a surgical anastomosis.

The magnetically hard connection portion and the magnetically soft connection portion differ by the response of their magnetization to an external magnetic field, wherein the magnetically hard connection portion may substantially act as a permanent magnet while the magnetization of the magnetically soft connection portion may substantially follow externally applied fields, e.g. feature a low magnetic hysteresis.

In preferred embodiments, the magnetic coercitivity of the magnetically soft connection portion is smaller than 1 kA/m.

The magnetically soft connection portion may be made of a ferromagnetic material for magnetically coupling to the magnetically hard connection portion, and the magnetic coercitivity of the magnetically soft connection portion should be lower than 1 kA/m, in particular 0,6 kA/m or lower, for enabling a reversal of the polarization of the magnetically soft connection portion with a local electromagnet. Suitable magnetically soft connection portions may include iron, cobalt, nickel or alloys thereof, such as iron with a low carbon content, FeCo, FeNi (permalloy), silicon steel, or may comprise soft ferrites or soft magnetic composites, such as iron based powder cores.

In preferred embodiments, the magnetic coercitivity of the magnetically hard connection portion is greater than 1 kA/m.

When the magnetic coercitivity of the magnetically hard connection portion is larger than 1 kA/m, in particular larger than 10 kA/m or larger than 100 kA/m, the magnetization of the magnetically hard connection portion may be substantially permanent, therefore resulting in a reliable implant. The magnetically hard connection portion should be magnetically polarized, e.g. along the extension direction of the through-going passage, to magnetically attract the magnetically soft connection portion of the first clamping element. Due to the comparatively large coercitivity, the magnetically hard connection portion may retain its magnetization when the magnetization of the magnetically soft connection portion is reversed. The magnetically hard connection portion may be formed by a permanent magnet, such as a permanent magnet based on magnetically hard iron alloys (e.g. an AlNiCo magnet), hard ferrites, SmCo based alloys, or NdFeB based alloys. Preferably, the magnetically hard connection portion comprises an NdFeB magnet, which is most preferably polarized along the extension direction of the through-going passage.

In preferred embodiments, a relative magnetic permeability of the magnetically soft connection portion is larger than 50, in particular larger than 300, preferably larger than 1000.

Preferably, the relative magnetic permeability of the magnetically soft connection portion is larger than 300, such as 10³ or 10⁴, or larger, to increase a magnetic attraction between the magnetically soft connection portion and the magnetically hard connection portion.

The magnetically soft connection portion and the magnetically hard connection portion may be integrated into a clamping body of the first clamping element and the second clamping element, respectively, for distributing the clamping forces around the central through-going passage. In other words, each of the first clamping element and the second clamping element may comprise a clamping body comprising the magnetically soft connection portion and the magnetically hard connection portion, respectively, and defining the perimeter of the central through-going passage through the clamping elements. The clamping body may circumscribe the through-going passage in a closed loop. The magnetically soft connection portion and the magnetically hard connection portion may be housed in the clamping body and/or portions of the clamping body may have magnetically soft/hard material sections. For example, the clamping body of the second clamping element may hold one or more permanent magnets in a relative connection geometry and the first clamping element may comprise magnetically soft material portions for joining the first clamping element and the second clamping element.

In preferred embodiments, the first clamping element comprises a plurality of magnetically soft connection portions and the second clamping comprises a plurality of magnetically hard connection portions, wherein the plurality of magnetically soft connection portions and the magnetically hard connection portions are arranged circumferentially around the common through-going passage of the first clamping element and the second clamping element, respectively, to magnetically couple the first clamping element and the second clamping element.

For example, the clamping elements may comprise a plurality of connection segments circumferentially arranged around the common through-going passage and associated with respective magnetically soft/hard connection portions. The arrangement of the plurality of magnetically soft connection portions may correspond to the plurality of magnetically hard connection portions, e.g. the plurality of magnetically soft connection portions of the first clamping element may be in an arrangement mirroring the arrangement of the plurality of magnetically hard connection portions of the second clamping element.

The plurality of magnetically soft connection portions may be targeted by individual electromagnets. For example a plurality of electromagnets may each be spatially associated to one of the magnetically soft connection portions and may be configured for targeting each of the magnetically soft connection portions at the same time. Individually targeting a plurality of magnetically soft connection portions may be preferable, e.g. may be associated with an improved magnetic coupling, with respect to targeting a single (e.g. ring-shaped) soft connection portion for disconnecting the clamping elements from each other.

The plurality of magnetically soft/hard connection portions may be distributed substantially regularly around the center through-going passage, such as to distribute compressive/clamping forces by the clamping device onto the flexible tubular walls around the perimeter of the central through-going passage.

In preferred embodiments, the first clamping element comprises a magnetic coupling section for magnetically coupling to the magnetically soft connection portion at a coupling side, wherein the coupling side is different from a joining side for coupling the first clamping element and the second clamping element to each other, in particular opposite the joining side.

The first clamping element may be configured to be joined to the second clamping element at the joining side, and may be configured to be coupled to an external electromagnet at the coupling section. Preferably, the first clamping element is configured such that a magnetic coupling of the magnetically soft connection portion to an external magnet at the coupling section is equal to or greater than the magnetic coupling of the magnetically soft connection portion to an external magnet at the joining side. When the magnetic coupling at the coupling section to the external (electro-)magnet is larger than the coupling to the magnetically hard connection portion of the second clamping element, the reversal of the magnetization of the magnetically soft connection portion may be facilitated. For example, a coating material around the magnetically soft connection portion, such as a polymer enclosure of a clamping body, may have a thickness at the coupling section which is equal to or smaller than a thickness of the coating material covering the magnetically soft connection portion at the joining side.

In preferred embodiments, the magnetic coupling section comprises a protruding portion of the magnetically soft connection portion, wherein the protruding portion protrudes away from the joining side of the first clamping element.

For example, a protruding portion of the magnetically soft connection portion may form a coupling pin protruding away from the joining side of the first clamping element. The coupling pin may be received in an electromagnet, e.g. in the space surrounded by coils of the electromagnet, to increase a coupling between the magnetically soft connection portion and the electromagnet and to facilitate the reversal of the magnetization of the magnetically soft connection portion, while the magnetically soft connection portion is magnetically coupled to the magnetically hard connection portion of the second clamping element.

Additionally or alternatively, the magnetic coupling section may comprise a recess protruding towards, and in particular into, the magnetically soft connection portion for receiving a magnetic coupling portion of an applicator.

The recess may be configured to receive a protruding portion of the core of an external electromagnet. When the core of the electromagnet protrudes towards, and in particular into, the magnetically soft connection portion, the coupling between the electromagnet and the magnetically soft connection portion of the first clamping element may be increased, such as to facilitate the reversal of the magnetization of the magnetically soft connection portion while the magnetically soft connection portion is magnetically coupled to the magnetically hard connection portion of the second clamping element.

The recess and/or the protruding portion of the first clamping element may be tapered, such as to promote a self-alignment of an applicator and the coupling section of the first clamping element, e.g. for disconnecting the clamping device from joined bowel sections. For example, the recess of the first clamping element may be partially cone-shaped, e.g. close to the periphery of the first clamping element, such that a rod-shaped magnet core protruding from the coils of an electromagnet is guided towards a comparatively narrower center of the recess for coupling the magnetic core to the magnetically soft connection portion. Similarly, the protruding portion of the first clamping element may comprise a tapered end, such as a rounded or cone-shaped tip, to allow partial self-alignment of the protruding portion with a recess associated with the coils of an external electromagnet.

In preferred embodiments, the first clamping element and/or the second clamping element comprise(s) internal suction conduits to apply a suction force at a joining side of the first clamping element and/or the second clamping element for holding an end section of one of the flexible tubular walls to the joining side.

During transluminal application of the clamping device, the flexible tubular walls should be held on facing joining sides of the first clamping element and the second clamping element for clamping the flexible tubular walls between the clamping elements. The flexible tubular walls, e.g. tissue sections, may be held over the joining sides of the first and second clamping elements through the suction force applied through the suction conduits. Hence, in operation, the flexible tubular walls may be folded over the clamping elements and held in place by applying a vacuum force at an opposite side of the clamping elements which may result in a clamping device which is easy to use. The suction conduits may connect different faces of the clamping devices, such that a vacuum may be applied at one face of the clamping devices to induce a suction force at the joining side of the clamping devices.

In preferred embodiments, the first clamping element and the second clamping are each convertible between a first and a second configuration to change a perimeter of the common through-going passage.

A selectable perimeter may facilitate the deployment of the clamping device and may enable the creation of a customized surgical anastomosis which can be adapted to the anatomical/physiological conditions of a patient. In particular, the first clamping element and the second clamping element may be converted from the first configuration to the second configuration during a surgical procedure, such as to facilitate the transluminal approach of the implant to the luminal margins and to simplify the following surgical procedure. For example, the first clamping element and the second clamping element may be transluminally approached to the anatomical site of resection in a first configuration featuring a small perimeter and may then be expanded to the second configuration featuring a larger perimeter than the first configuration. In addition, an applicator may be retracted through the central through-going passage after the clamping device has been expanded towards the larger perimeter.

The perimeter may be adjusted by radially expanding each of the convertible clamping elements along a radial direction, such that the surrounding tissue wall of respective first and second lumina may fold over joining surfaces of the clamping elements and may be aligned for the creation of a surgical anastomosis; the radial direction being perpendicular to the through-going passage defined by the clamping elements.

Preferably, the perimeter should be adjustable by adjusting a spacing between adjacent magnetically soft/hard connection portions of the first clamping element and of the second clamping element, respectively.

The first clamping element and the second clamping element should maintain their perimeters in the second configuration in the absence of external forces. By maintaining the established perimeter of the surgical anastomosis, a uniform healing process of the tissue sections may be stimulated while leakage may be prevented. Preferably, the first clamping element and the second clamping element are convertible for selecting and maintaining the inside perimeter of the first clamping element and the second clamping element, respectively, when the clamping device is deployed for surgical anastomosis in order to establish the perimeter of the through-going passage. After the clamping device has been deployed for creating the surgical anastomosis, an applicator may be retracted through the expanded central through-going passage of the clamping device.

The skilled person will appreciate that a limited degree of flexibility can nonetheless be desirable to support the natural function of the tissue at the surgical anastomosis. Hence, the maintained perimeter in the absence of external forces should be understood to not exclude elastic deformation of the first clamping element and the second clamping element when the implant is deployed for surgical anastomosis. Rather, the maintained perimeter should be understood to constitute a target perimeter which can be selected by converting the first clamping element and the second clamping element between the first configuration and the second configuration, while the first clamping element and/or the second clamping element may feature a restoring force biasing the first clamping element and/or the second clamping element towards the maintained (target) perimeter. However, the first clamping element and/or the second clamping element may also be rigid to prevent deformation of the surgical anastomosis after converting the clamping element(s) between the first configuration and the second configuration.

In addition, the skilled person will appreciate that the first clamping element and the second clamping element may be converted back and forth between different configurations featuring different perimeters during a (surgical) intervention, such as to determine a suitable implantation configuration. For example, the clamping device may be expanded for folding tissue walls over the clamping elements, and the clamping elements may subsequently be at least partially collapsed for connecting the matching connection elements. The clamping elements may subsequently be (re-)expanded to the suitable implantation configuration for establishing the through-going passage. The clamping elements may then maintain the perimeter of the through-going passage, when the clamping device is deployed for surgical anastomosis.

Additionally or alternatively, the perimeter and/or the shape of the clamping elements may be passively adjusted following deployment over a transitory period, such as by swelling or by deflating of the clamping elements due to an interaction with the surrounding environment. For example, absorbing components or biodegradable components of the clamping elements may progressively absorb body liquids or may be progressively degraded, respectively, such as to adapt to tissue swelling or deswelling at the anatomical site of the intervention. In some embodiments, the clamping device substantially maintains the perimeter of the through-going passage over a transitory period during a healing process of the surrounding tissue sections.

Preferably, the first clamping element and/or the second clamping element are convertible to adjust a spacing of a plurality of connection elements along the perimeter of the respective clamping element from the first configuration towards the second configuration and to lock the relative spatial configuration and/or the spacing of the connection elements in the second configuration, such that the locked relative spatial configuration and/or the locked spacing of the connection elements maintains the perimeter of the first clamping element and/or the second clamping element. Hence, the clamping device may be expanded at the anatomical site of the intervention while the connection elements maintain a relative alignment along the perimeter of the respective clamping element for creating the surgical anastomosis. Each of the connection elements of the first clamping element and the second clamping element may comprise a magnetically soft/hard connection portion, respectively.

In some embodiments, the first clamping element and the second clamping element can be stored in a transport configuration to be transluminally approached to the anatomical site of the intervention and can then be expanded to a deployed configuration, wherein a perimeter of the respective clamping element in the deployed configuration can be selected from a range of supported perimeters and wherein the first clamping element and the second clamping element maintain the selected perimeter in the absence of external forces. For example, the perimeter of the first clamping element and the second clamping element may be expanded, such that a diameter of the through-going passage is increased by at least 10%, preferably by at least 20%, such as by 30%.

In some embodiments, the first clamping element and the second clamping element are configured to transfer and/or to distribute compressive forces on body tissue held between the first clamping element and the second clamping element, such that a healing process of the body tissue is stimulated along a closed loop around the perimeter of the through-going passage when the clamping device is deployed for supporting a surgical anastomosis.

Hence, the clamping device may induce and support a surgical anastomosis and may prevent leakage from the site of the anatomical intervention. The body tissue held between the first clamping element and the second clamping element may grow together to form the surgical anastomosis. The clamping device may be removed from the surgical anastomosis when the body tissue sections of the first and second lumina adhere to each other. In some embodiments, the clamping elements may be configured to collapse after a predetermined period of time and may be excreted. For example, the clamping elements may feature a predetermined breaking point formed of a biodegradable material and/or may be filled with a biocompatible material which maybe (progressively) leaked, degraded or absorbed, such that the clamping elements separate from the surrounding body tissue after a predetermined period of time when the structural integrity of the clamping elements is sufficiently reduced.

When the clamping device is deployed for surgical anastomosis, the holding force holding the first clamping element against the second clamping element by the matching connection elements may also overcome a threshold for inducing necrosis in at least portions of the clamped tissue sections arranged between (axial surfaces of) the first clamping element and the second clamping element. Tissue sections joined by the clamping elements may then adhere to each other and grow together for forming the surgical anastomosis while clamped tissue sections compressed between (axial surfaces of) the clamping elements may necrotize and subsequently separate from the surrounding tissue. The clamping device may then separate from the surrounding tissue by release of the necrotic tissue and may be excreted with the separating necrotized tissue clamped by the clamping elements.

In some embodiments, the first clamping element and the second clamping element comprise a clamping body circumscribing the through-going passage in a closed loop, wherein the clamping body defines the perimeter in the first configuration and in the second configuration.

The continuous clamping body circumscribing the through-going passage in a closed loop may apply uniform pressure from both sides to clamped tissue portions. The uniform pressure may limit leakage from the surgical anastomosis and may also induce a uniform healing process along the closed loop. In addition, necrosis may be induced through the clamping pressure in the closed loop, such that the clamping device may reliably separate from the surgical anastomosis.

The matching connection elements should be fixedly attached to the clamping body. With the matching connection elements fixedly attached to the clamping body, the number of manipulated components of the clamping device can be limited and the application of the clamping device can be facilitated.

In some embodiments, the first clamping element and the second clamping element each comprise a plurality of rigid sections interconnected by adjustable joining sections, wherein the first clamping element and the second clamping element are convertible between the first configuration and the second configuration by adjusting the joining sections to establish a different spacing between the plurality of rigid sections in the first configuration and in the second configuration.

The rigid sections and the joining sections may mutually form the clamping body circumscribing the through-going passage in a closed loop. The different spacing of the rigid sections may adjust the perimeter of the first clamping element and the second clamping element for converting the first clamping element and the second clamping element between the first configuration and the second configuration.

The rigid sections may each comprise magnetically soft/hard connection portions as connection elements. Hence, an applicator may mount the clamping device via the rigid sections associated with the connection elements to define a relative connection geometry, while adjustment of the joining sections may enable an operator to select a perimeter of the first clamping element and the second clamping element to establish a perimeter of the though-going passage when the clamping device is deployed for anastomosis.

The joining sections should be configured to maintain the spacing between the plurality of rigid sections when the perimeter of the through-going passage is established.

In some embodiments, the joining sections comprise a shape setting element, in particular a convertible cavity for receiving a filling medium and/or a locking mechanism and/or a compressible elastic element, to establish the perimeter of the through-going passage.

The shape setting element may adjust and maintain a spacing of the rigid sections to set a perimeter of the first clamping element and the second clamping element.

The convertible cavity may be filled with the filling medium when the clamping device is deployed for surgical anastomosis, such that the convertible cavity is expanded by the filling medium to adjust the spacing between the rigid sections. For example, the convertible cavity may comprise a flexible conduit connecting the rigid sections and may be expandable to adjust the spacing between the rigid sections. The joining sections may be coupled to a manipulation port of the respective clamping element configured to receive the filling medium and to guide the filling medium to the convertible cavity. The filling medium filling the convertible cavity may maintain the spacing of the rigid sections by preventing a collapse of the convertible cavity.

The filling medium may comprise polymerizing solutions or gels for maintaining the spacing of the rigid sections with crosslinked polymers, such as gels or solutions of superabsorbent polymers, e.g. a Hydrogel, orthophosphates and/or epoxides, which can be injected into the convertible cavity for expanding the joining sections and for maintaining a continuously adjustable spacing between the rigid sections. The clamping elements may comprise a liquid port to connect to a fluid conduit for receiving the filling medium. The liquid port may be associated with a valve element for preventing a discharge of the filling medium from the convertible cavity. In some embodiments, the filling medium hardens for maintaining the spacing between the rigid sections after expansion of the convertible cavity and for preventing a discharge of the filling medium from the convertible cavity. In addition, absorbing or hardening additives may be provided in cavities of the clamping elements to absorb body liquids and to passively adjust a shape of the clamping device or compressive force on the tissue sections after implantation.

The filling medium may harden via a polymerization process, such as the hardening process of bone cement, or may harden by fluid absorption and a corresponding viscosity increase, as in the case of a hydrogel. Hence, in the context of the clamping elements the term "hardening" should be understood broadly to refer to a change of the elastic properties of the filling medium after the filling medium has been filled into the convertible cavity, such that the clamping elements may establish the perimeter of the through-going passage, not limited to a polymerization process or phase transition.

The clamping elements may comprise a convertible cavity forming a closed loop around the through-going passage, and the rigid sections may comprise internal fluid connections to connect different sections of the convertible cavity. In some embodiments, the clamping elements comprise a plurality of convertible cavities arranged between the rigid sections wherein the convertible cavities each are connectable to fluid conduits for individually receiving the filling medium.

In some embodiments, the rigid sections may be reinforced sections of an elastic clamping body comprising a convertible cavity, wherein the matching connection elements may be attached to the reinforced sections.

For example, the clamping elements may comprise an elastic and at least partially hollow clamping body, such as an elastic tube which may be reinforced by elastic biasing elements, e.g. an elastic spiral element, and the elastic clamping body may feature reinforced sections associated with the matching connection elements. The clamping elements may then be deployed by expanding the clamping elements via a restoring force of the elastic clamping body from a storage/transport configuration towards an expanded configuration featuring a larger perimeter than the storage/transport configuration. The clamping elements may be adjusted via further expansion of the convertible cavity, e.g. by filling the convertible cavity with the filling medium.

The elastic biasing elements may be configured to provide different elastic moduli along different spatial dimensions when deformed by the filling medium, such that the force imparted by the filling medium asymmetrically adjusts the thickness and the perimeter of the clamping elements. For example, the thickness of the clamping elements may remain substantially constant while the perimeter of the clamping elements is adjusted via the filling medium, or vice-versa, or a thickness of the clamping elements may be adjusted substantially along the axial direction of the clamping elements, i.e. along the through-going passage, and/or the radial direction when the clamping elements are filled with the filling medium. Additionally or alternatively, the filling medium or absorbing/hardening additives arranged in the clamping elements may swell to expand the clamping elements following implantation, such as to progressively expand the clamping elements following deployment and/or to passively adapt the clamping device to the anatomical conditions. For example, the clamping elements may be configured to passively adjust the shape towards an anatomically possible perimeter and/or to compensate for a subsiding postoperative tissue swelling along the radial direction and/or the axial direction.

Alternatively or additionally, the joining sections may comprise a locking mechanism convertible between different configurations to mechanically adjust and lock a spacing between the rigid sections. For example, the locking mechanism may comprise two interlocking sections respectively connected to adjacent rigid sections, such as a locking pin attached to a first rigid section and a rack featuring form-locking recesses attached to a second rigid section, wherein the pin may selectively interlock with the form-locking recesses to adjust and lock a spacing between the rigid sections. In some embodiments, the locking mechanism is configured to allow relative movement between the rigid sections in an expansion direction and to prevent relative movement in the opposite direction, e.g. by comprising asymmetrically formed interlocking sections, such that the clamping element may be expanded during deployment but prevents a subsequent collapse of the through-going passage after the target perimeter has been established.

Alternatively or additionally, the clamping elements may comprise a compressible elastic element to bias the clamping element towards a target perimeter when the clamping device has been deployed for surgical anastomosis. For example, the joining sections may comprise spring elements for insertion into a lumen, which are released to irreversibly expand the clamping element towards a target shape/perimeter. In some embodiments, the spring elements comprise a shape-memory alloy which is cooled below a transformation temperature and compressed for approaching the anatomical site of the intervention. Upon heating, the shape-memory alloy may cross the transformation temperature to bias the spring element towards a larger perimeter. Hence, the clamping elements may be expanded without an external mechanical force (e.g. cable pull transmitted forces, pneumatic forces or hydraulic forces) or magnetic force by the biasing force of the compressible elastic element. In some embodiments, the clamping body is elastic and is biased to assume an expanded shape, e.g. the clamping body may comprise an elastic hollow tube section featuring a spiral spring integrated with the elastic hollow tube section, for biasing the clamping body towards an expanded diameter.

In some embodiments, the first clamping element and the second clamping element further comprise mounting elements to mount the first clamping element and the second clamping element to an applicator on the inner perimeter of the first clamping element and the second clamping element, respectively.

The mounting elements may be arranged on the inner perimeter of the first clamping element and/or the second clamping element or may be arranged on the side surfaces adjacent to the inner perimeter of the first clamping element and/or the second clamping element, in particular on a side opposite of the matching connection elements, such as to mate with a matching mounting support on an outer face of an applicator. The mounting elements may feature clamping brackets to engage with a clamping element of the applicator, such as to mechanically mount the first clamping element and/or the second clamping element on the applicator.

In some embodiments, the clamping elements may be mountable to an applicator via the magnetically soft/hard connection portions.

The mounting elements may hold the first clamping element and the second clamping element on the outer face of the applicator during an approach of the implant towards the anatomical site of the intervention and during an alignment of the respective tissue walls over the axial facing surfaces of the clamping elements. When the first clamping element is connected to the second clamping element, the applicator may be disconnected from the clamping elements and may be retracted through the through-going passage formed by the implant. In some embodiments, a radial displacement of the mounting supports of the applicator can be used to expand the perimeter of the through-going passage and to convert the first clamping element and the second clamping element between the first configuration and the second configuration. Hence, the implant may be simple to use and to deploy.

In addition, manipulation ports of the clamping elements, such as fluid ports for receiving a filling medium or mechanical actuators, to convert the first clamping element and the second clamping element between the first configuration and the second configuration may be integrated with and/or arranged in a fixed spatial relationship with the mounting elements, such as to control the perimeter of the first configuration and the second configuration via applicator elements attached to the mounting support. The perimeter of the implant may then be reliably changed through the applicator elements while the first clamping element and the second clamping element is mounted to the applicator via the mounting elements, even if the position of the mounting supports is changed in accordance with the change of the configuration of the clamping elements.

In a second aspect, the invention relates to an applicator for manipulating a clamping device with a first clamping element and a second clamping element. The first clamping element and the second clamping element comprise a magnetically soft connection portion and a magnetically hard connection portion, respectively. The applicator comprises an applicator body, a first manipulator and a second manipulator mounted on the applicator body, and a control system. The first manipulator and the second manipulator are configured to be displaced with respect to each other along the applicator body. The first manipulator and the second manipulator are configured to hold the first clamping element and the second clamping element, respectively, in an arrangement in which the first clamping element and the second clamping element are facing each other. The first manipulator comprises an electromagnet for coupling to the magnetically soft connection portion of the first clamping element. The control system is configured to selectively apply a separation current profile to the electromagnet of the first manipulator to reverse a magnetization of the magnetically soft connection portion of the first clamping element while the first clamping element is magnetically coupled to the second clamping element, such that the first clamping element and the second clamping element are separated from each other through magnetic forces. The applicator can be used to deploy a clamping device according to the first aspect transluminally, i.e. through and from the inside of a flexible lumen, to clamp respective portions of the flexible tubular walls together. The electromagnet of the first manipulator can be driven to magnetically manipulate the clamping elements, i.e. by generating repelling and attracting forces onto the magnetic/magnetizable portions of the clamping elements.

The electromagnet of the first manipulator is adapted to generate a magnetic field to separate the first clamping element and the second element when they adhere to each other through magnetic forces. In other words, the electromagnet of the first manipulator should be configured to generate a local magnetic field at the mounting position of the first clamping element on the first manipulator with a magnitude and/or temporal profile that is sufficient to reverse the magnetization of the magnetically soft connection portion while it is coupled to the magnetically hard connection portion. The applicator may be configured to deploy the clamping device in an anatomical lumen for creating a surgical anastomosis as an implant applicator, and the generated local magnetic field should therefore be suitable to reverse the magnetization of the magnetically soft connection portion of an implant that is suitable to create a surgical anastomosis while the magnetically soft and magnetically hard connection portions are joined to each other.

In preferred embodiments, the first manipulator and the second manipulator each comprise an electromagnet for coupling to the magnetically soft connection portion of the first clamping element and to the magnetically hard connection portion of the second clamping element, respectively.

When each of first manipulator and the second manipulator comprises an electromagnet, the clamping elements may be selectively mounted to either one of the manipulators through magnetic forces to facilitate manipulation of the clamping elements.

In preferred embodiments, the electromagnet of the first manipulator comprises a magnetic core exposed towards the magnetically soft connection portion of the first clamping element and is configured to magnetically couple to the magnetically soft connection portion of the first clamping element.

The magnetic core may be magnetically soft and may be made of a ferromagnetic material. The magnetic core may be aligned and exposed along an axial direction of the applicator, e.g. along the extension of the applicator body, such that the magnetic core is exposed at an axial face of the first manipulator at which the first clamping element is mounted to enhance a magnetic coupling between the electromagnet and the clamping device. The skilled person will appreciate that the magnetic core may nonetheless be covered by a coating, such as to prevent exposure of the magnetic core to body fluids, but that said coating should only negligibly impact the magnetic coupling between the core and the magnetically soft connection portion, e.g. the coating spacing the magnetic core from the periphery of the manipulator towards the magnetically soft connection portion may have a thickness smaller than 2 mm or smaller than 1 mm. In some embodiments, the first manipulator may comprise a plurality of electromagnets, and a plurality of magnetic cores of the electromagnets, e.g. each of the magnetic cores of the electromagnets, may be exposed towards a corresponding plurality of magnetically soft connection portions of the first clamping element.

In some embodiments, the first manipulator comprises a mounting rail for holding the first clamping element in a rotationally fixed manner, such that the magnetically soft portion of the first clamping element is aligned in the circumferential direction with the electromagnet, e.g. by engaging with a corresponding recess or protrusion of the first clamping element.

In preferred embodiments, the first manipulator comprises a recess protruding towards, and in particular into, the electromagnet for receiving a protruding coupling portion of the first clamping element.

Accordingly, a protruding portion of the magnetically soft magnetic portion may protrude into the electromagnet, such as to lie inside of the space circumscribed by the coils of the electromagnet for increasing a magnetic coupling between the electromagnet and the magnetically soft connection portion.

Additionally or alternatively, the first manipulator may comprise a protruding portion magnetically coupled to the electromagnet, wherein the protruding portion protrudes from the electromagnet towards the second manipulator.

For example, a ferromagnetic core of the electromagnet may protrude towards the second manipulator to protrude towards and/or into a portion of the first clamping element for increasing a magnetic coupling between the electromagnet and the magnetically soft connection portion.

The recess and/or the protruding portion of the first manipulator may be tapered such as to promote a self-alignment of the first manipulator and the coupling section of the first clamping element, e.g. for disconnecting the clamping device from joined bowel sections. For example, the recess of the first manipulator may be partially cone-shaped, e.g. at the axial face of the first manipulator, such that a rod-shaped magnetizable portion protruding from the first clamping element is guided towards a narrow center of the recess for coupling the magnetic core of the first manipulator to the magnetically soft connection portion. Similarly, the protruding portion of the first manipulator may comprise a tapered end, such as a rounded or cone-shaped tip to allow partial self-alignment of the protruding portion with a recess of the first clamping element. The engagement of the respective recesses and protruding portions may also prevent a relative rotational movement between the clamping element and the manipulator around the through-going passage.

The control system may then be configured to drive the electromagnet of the first manipulator with a separation current profile to separate the first and second clamping elements.

The control system may comprise a single control unit or may comprise a plurality of control units which may be functionally connected. The control units may comprise a microcontroller, an ASIC, a PLA (CPLA), an FPGA, or other control devices, including control devices operating based on software, hardware, firmware, or a combination thereof. The control devices can include an integrated memory, or communicate with an external memory, or both, and may further comprise interfaces for connecting to sensors, devices, appliances, integrated logic circuits, other controllers, or the like, wherein the interfaces may be configured to receive or send signals, such as electrical signals, optical signals, wireless signals, acoustic signals, or the like.

The control system may comprise or control a current source and/or voltage source for generating the separation current profile and/or may control electrical switches to selectively apply a current from the current source and/or voltage source to the electromagnet and/or to select a polarity of the applied separation current profile.

In preferred embodiments, the control system is configured to selectively apply a first current having a first polarity to the electromagnet of the first manipulator in a first time slot and to selectively apply a second current having an opposite second polarity to the electromagnet of the first manipulator in a second time slot to separate the first clamping element and the second clamping element.

As an example, the control system may change between the first current and the second current with a switch changing the polarity of a driving current source. However, the skilled person will appreciate that the first and second currents may but do not need to have the same magnitude in embodiments.

Experiments of the inventors showed that the subsequent application of the first and second currents can separate a magnetically soft connection portion and a magnetically hard connection portion which were previously magnetically joined to each other. The inventors also observed that the application of the second current without applying the first current may repel the first and second clamping elements from the first manipulator, thereby separating the clamping device, i.e. both clamping elements, from the first manipulator.

It is believed that the first current can increase a coupling between the electromagnet of the first manipulator and the magnetically soft connection portion of the first clamping element by attracting the first and second clamping elements towards the first manipulator. The subsequent second current reverses the direction of the magnetic field generated by the electromagnet and can reverse the magnetization of the magnetically soft connection portion of the first clamping element. However, due to a comparatively larger magnetic adhesion force between the first clamping element and the electromagnet (e.g. due to a comparatively larger magnetization of the core of the electromagnet), the magnetically soft connection portion is not repelled during the reversal of the current polarity, but its magnetization is instead reversed.

Whether the applied separation current profile results in the separation of the first clamping element and the second clamping element may depend on the magnetic coupling strength between the electromagnet and the magnetically soft connection portion as well as the magnetic attraction between the clamping elements. The magnetic coupling strength between the magnetically soft connection portion and the electromagnet may be increased through the chosen geometry of the core of the electromagnet and the first clamping element, e.g. through physical engagement of the core and the soft magnetic connection portion, or the selected materials of the core and the magnetically soft connection portion.

In addition, the magnetic coupling strength between the core of the electromagnet and the first clamping element may also be increased at a first point in time by applying the first current, and the first and second clamping elements may be separated at a second non-subsequent point in time with the second current. Hence, in some embodiments, the first current is substantially zero in a time frame leading up to the application of the second current.

In some embodiments, the control system is configured to selectively ramp the current applied to the electromagnet of the first manipulator at most with a separation rate of change to separate the first clamping element and the second clamping element, wherein the first clamping element and the second clamping element are separated from the first manipulator when the current is ramped at a rate higher than the separation rate of change.

However, the reversal of the polarization may also be achieved by mechanically restricting a motion of the first clamping element.

In preferred embodiments, the first manipulator comprises a holding bracket to selectively retain the first clamping element along an axial direction of the applicator (e.g. along the applicator body).

Hence, the clamping elements of the clamping device may also be separated by retaining the first clamping element at the first manipulator, e.g. through a mechanical engagement of the first manipulator and the first clamping element, while the magnetization of the magnetically soft connection portion is reversed with the electromagnet, such as to prevent a repulsion of the electromagnet and the first clamping element while the magnetic field of the electromagnet is building up.

Accordingly, the clamping device may be disconnected from joint tissue sections without the application of comparatively large mechanical forces (with respect to the clamping force) along the applicator body.

The elongated applicator body should be configured for transluminal approach towards the tissue sections and may accordingly feature flexible or flexibly connected segments to navigate through an anatomical lumen, which may be selectively stiffened. In preferred embodiments, the applicator body comprises an accommodating passage to mount the applicator on the outer face of an endoscope. Hence, the applicator may be combined with the endoscope to navigate through an anatomical lumen. Additionally or alternatively, the applicator may comprise an imaging device configured to capture an image at/or close to the first manipulator and/or the second manipulator and/or to transmit an image from the distal portion of the applicator to a proximal portion of the applicator, such as to integrate the functionality of an endoscope with the applicator or to provide additional imaging capabilities, e.g. for supporting an operator while creating the surgical anastomosis.

In preferred embodiments, the outer diameter of the applicator is smaller than 60 mm, in particular smaller than 40 mm, preferably smaller than 30 mm in a navigation configuration of the applicator to navigate through an anatomical lumen. In the navigation configuration, the manipulator may be held against the elongated applicator body to minimize the outer circumference of the applicator while the anatomical lumen, e.g. a human colon, is navigated. Preferably, the outer diameter of the applicator is smaller than 40 mm, most preferably smaller than 30 mm, in the navigation configuration including the space occupied by the clamping elements mounted on the mounting supports.

In preferred embodiments, the applicator comprises a drive mechanism to move the first manipulator and/or the second manipulator with respect to the applicator body.

When the applicator approaches the anatomical site of the intervention with the manipulators, disconnected proximal and distal tissue sections, e.g. oral and aboral sections of the bowel, may be folded over the clamping elements on the outer perimeter of the applicator to align the respective proximal and distal tissue sections with respect to each other. The drive mechanism may then be driven to move the manipulators towards each other, thereby clamping the folded proximal and distal tissue sections between the clamping elements mounted on the manipulators. The tissue sections may be folded over the clamping elements and/or the manipulators with laparoscopic assistance and may be held against the clamping elements via sutures, clamps or hooks on the clamping elements or the manipulators.

In preferred embodiments, the drive mechanism comprises a fluid conduit for transmitting a hydraulic or pneumatic force to the first manipulator and/or the second manipulator, and/or a cable pull to transmit a mechanical force to the first manipulator and/or the second manipulator.

The mechanical force may be for approaching the first manipulator and the second manipulators towards each other and/or for spacing the first manipulator and the second manipulator away from each other.

The inventors also found that applying a mechanical separation force to the first and second manipulators while the magnetization of the magnetically soft connection portion of the first clamping element is reversed with the electromagnet of the first manipulator may increase a reliability of the separation process.

In preferred embodiments, the first manipulator and/or the second manipulator are convertible between a first configuration and a second configuration to deploy the clamping device in the first configuration and to retract the applicator through a central through-going passage of the clamping device in the second configuration.

For example, the applicator may be advanced through the flexible tubular wall, e.g. the aboral portion of a patient's bowel, in the second configuration and may be expanded towards the first configuration at a deployment location of the clamping device, such as to expand the clamping device. After deploying the clamping device at the deployment location, the first manipulator and/or the second manipulator may be converted back to the second configuration to be retracted through the central through-going passage.

However, in some embodiments, the applicator may be advanced through the flexible tubular wall in the first configuration and only be converted to the second configuration following the deployment of the clamping device.

In some embodiments, the pair of manipulators comprises pivotally hinged manipulator arms to pivot with respect to the elongated applicator body such that the tissue folding section is radially displaced away from the elongated applicator body to fold the tissue of the surrounding lumen.

The pivotal motion of the manipulator arms may be driven with any suitable mechanical drive mechanism, such as a cable pull connected to the applicator or may be driven with hydraulic forces applied through fluid conduits from a proximal side of the applicator, to actuate the pivot mounting of the pair of manipulators on the applicator. For example, a worm gear mounting of the manipulator may be driven by rotating or displacing a worm shaft on the elongated applicator body to drive a pivoting motion of the manipulator arms coupled to the worm shaft by a worm wheel. As another example, a displaceable runner on the elongated applicator body may be driven to pivot the pivotally mounted manipulators via stretchers similar to the opening mechanism of an umbrella.

The pivotal motion may radially displace portions of the clamping elements mounted on the manipulators away from the elongated applicator body, such as to radially expand the clamping elements in accordance with the motion of the manipulator arms.

While the manipulator arms are radially displaced, the surrounding tissue sections may be folded over the clamping elements via an elastic restoring force of the tissue. Preferably, the tissue sections are held in place on the manipulators while the drive mechanism approaches the pair of manipulators towards each other.

In preferred embodiments, the applicator further comprises suction conduits to apply a suction force at the first manipulator and/or the second manipulator.

The suction force may fold and/or reliably hold the tissue folded over the clamping element(s) and may be externally applied at a proximal end of the applicator via the suction conduits. Preferably, a suction port of the manipulator(s) is arranged facing suction conduits of the clamping element(s), such that the suction force may be applied at the joining side(s) of the clamping element(s) for holding the tissue sections over the clamping elements while joining the clamping elements and creating of the surgical anastomosis.

In preferred embodiments, the applicator further comprises fluid conduits to transfer a fluid to a cavity of each of the clamping elements, and/or an actuation assembly coupled to the manipulators and configured to change a radial distance of the electromagnets on the manipulators to the elongated applicator body by mechanical displacement of the pair of manipulators.

For example, the clamping elements may be mounted on an outer face of the manipulators and a pivoting motion of the manipulators may radially expand the clamping elements mounted on the manipulators. The radial motion may induce a change of the inner perimeter of the clamping element while a self-locking mechanism of the clamping element may maintain the inside perimeter when the implant is deployed for surgical anastomosis. Additionally or alternatively, a filling medium may be transferred to the cavity of each clamping element to inflate the clamping elements. In some embodiments, the applicator further features an actuator to drive a locking mechanism of the clamping elements, such as by driving a motion of a locking pin to lock a spatial configuration of the clamping element. When the clamping elements have been expanded, the respective mechanisms may be disconnected from the clamping elements, such as by disconnecting the fluid conduit from a corresponding fluid port of the clamping element.

In a third aspect, the invention relates to a system of a clamping device according to the first aspect and an applicator according to the second aspect, wherein the applicator of the second aspect is configured for manipulating the clamping device of the first aspect.

In a fourth aspect, the invention relates to a system comprising a clamping device and an applicator for manipulating the clamping device. The clamping device comprises a first clamping element and a second clamping element comprising a magnetically soft connection portion and a magnetically hard connection portion, respectively. The applicator comprises an applicator body, a first manipulator and a second manipulator mounted on the applicator body and configured to be displaced with respect to each other along the applicator body. The first manipulator and the second manipulator are configured to hold the first clamping element and the second clamping element, respectively, and the first manipulator comprises an electromagnet for coupling to the magnetically soft connection portion of the first clamping element.

Preferably, the first manipulator and the second manipulator each comprise one or more electromagnet(s) for coupling to the magnetically soft connection portion of the first clamping element and to the magnetically hard connection portion of the second clamping element, respectively.

The clamping device may comprise any one of the features of the clamping device according to the first aspect and the applicator may comprise any one of the features of the applicator according to the second aspect, wherein the applicator of the second aspect is configured for manipulating the clamping device of the first aspect.

In a fifth aspect, the invention relates to the use of the clamping device according to the first aspect as an implant and/or the applicator according to the second aspect in an intervention for creating a surgical anastomosis.

In a sixth aspect, the invention relates to a method of driving an applicator for a clamping device with a pair of separable first and second clamping elements having magnetically soft and magnetically hard connection portions, respectively. The method comprises applying a current having a first current polarity at a first electromagnet of the applicator while the first clamping element is held at the first electromagnet, and applying a current having an opposite second current polarity at the first electromagnet of the applicator for separating the first clamping element and the second clamping element.

Applying the current having the first current polarity at the first electromagnet may hold the first clamping element and the second clamping element at the first electromagnet, magnetically coupled to each other.

While the aspects of the invention have been described above primarily with respect to an application in a surgical intervention, the skilled person will appreciate that the clamping device and the applicator may also be dimensioned and configured to also be applied in different (non-chirurgical) application environment, which may place lower restrictions on the possible dimensions and materials of the respective devices.

### DETAILED DESCRIPTION OF EMBODIMENTS

The features and numerous advantages of the clamping device and corresponding applicator according to the present invention will best be understood from a detailed description of preferred embodiments with reference to the accompanying drawings, in which:
- Fig. 1A, 1B: schematically illustrate an example of an implant for creating a surgical anastomosis;
- Fig. 2A, 2B: schematically illustrate an example of an implant and corresponding applicator for creating a surgical anastomosis;
- Fig. 3A, 3B: schematically illustrate another example of an implant and corresponding applicator for creating a surgical anastomosis;
- Fig. 4A-4D: schematically illustrate the electrical manipulation of magnetic portions of clamping elements with electromagnets according to different exemplary configurations; and
- Fig. 5: schematically illustrates an example of a convertible implant and corresponding applicator for creating a surgical anastomosis.

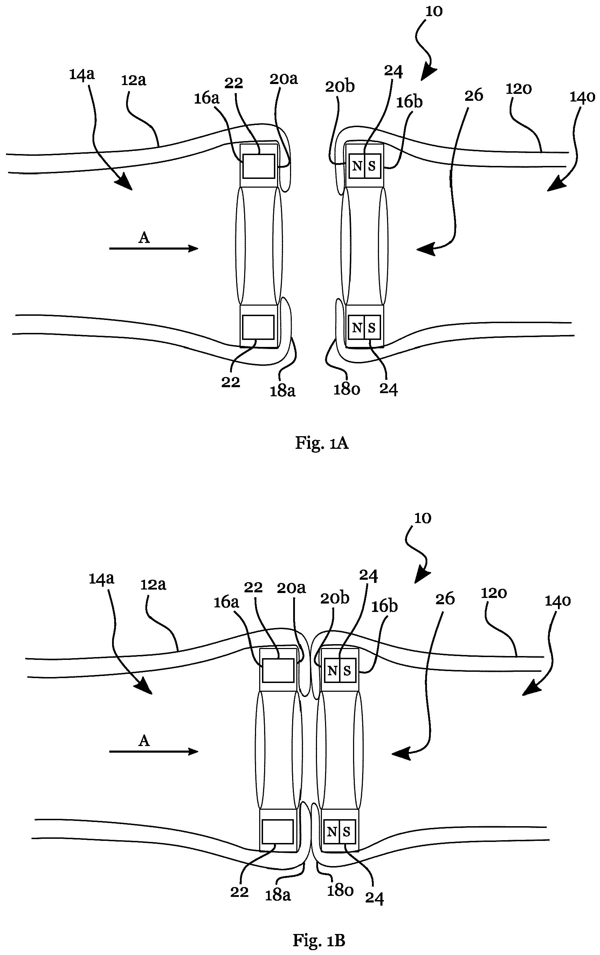

Fig. 1A and 1B schematically illustrate an example of a clamping device configured as an implant 10 for creating a surgical anastomosis depicted in the context of surgically disconnected bowel sections 12a, 120. The disconnected bowel sections 12a, 120 consist of an aboral tissue section 12a and an oral tissue section 120 forming an aboral lumen 14a and an oral lumen 140, respectively. The implant 10 comprises a first clamping element 16a and a second clamping element 16b. The first clamping element 16a is located in the aboral lumen 14a and the second clamping element 16b is located within the oral lumen 140.

In Fig. 1A folded tissue sections 18a, 180 of the aboral tissue section 12a and the oral tissue section 120 are folded over facing surfaces 20a, 20b of the first clamping element 16a and the second clamping element 16b, respectively. The facing (axial) surfaces 20a, 20b are aligned with each other, such that the folded tissue sections 18a, 180 of the aboral tissue section 12a and the oral tissue section 120 are substantially aligned in parallel and face each other along the axial direction A.

In Fig. 1B, the first clamping element 16a and the second clamping element 16b are moved towards each other, such that the folded tissue sections 18a, 180 of the aboral tissue section 12a and the oral tissue section 120 are clamped to each other by the facing surfaces 20a, 20b of the first clamping element 16a and the second clamping element 16b located on opposite sides of the respective folded tissue sections 18a, 180.

To join the first clamping element 16a and the second clamping element 16b to each other, the first clamping element 16a comprises a magnetically soft ferromagnetic portion 22 and the second clamping element 16b comprises a permanent magnet 24. The permanent magnet 24 is magnetically hard, i.e. features a coercitivity greater than 1 kA/m, in particular greater than 100 kA/m, and may be polarized along the axial direction A (as indicated by the magnetic poles "N" and "S"). The magnetically soft ferromagnetic portion 22 features a coercitivity smaller than 1 kA/m and may therefore be polarized in a comparatively low magnetic field.

The ferromagnetic portion 22 and the permanent magnet 24 are arranged at mirroring positions of the first clamping element 16a and the second clamping element 16b, respectively, such that the ferromagnetic portion 22 can be magnetically polarized by the permanent magnet 24, when the first clamping element 16a and the second clamping element 16b approach each other.

In the joined configuration illustrated in Fig. 1B, the polarized ferromagnetic portion 22 and the permanent magnet 24 may attract each other via magnetic forces and may hold the first clamping element 16a and the second clamping element 16b in a fixed relative spatial relationship while the surgical anastomosis can be created by a healing process of joined tissue sections 12a, 120 surrounding the implant 10.

Each of the clamping elements 16a, 16b features a clamping body circumscribing a through-going passage 26 in a closed loop. When the implant 10 is deployed for surgical anastomosis as schematically illustrated in Fig. 1B, the first clamping element 16a and the second clamping element 16b mutually form a through-going passage 26 connecting the aboral lumen 14a and the oral lumen 140 in order to restore the natural function of the bowel.

The ferromagnetic portion 22 and/or the permanent magnet 24 may be ring-shaped to circumscribe the through-going passage 26. However, in preferred embodiments, a plurality of ferromagnetic portions 22 and/or a plurality of permanent magnets 24 is arranged around the through-going passage 26 in the first clamping element 16a and the second clamping element 16b, respectively, in order to jointly hold the clamping elements 16a, 16b to each other.

The implant 10 may in principle be installed via conventional surgical methods, such as open surgery or laparoscopic surgery. However, the implant 10 is preferably configured for transluminal application to be approached towards the resected bowel section via the aboral lumen 14a or the oral lumen 140.

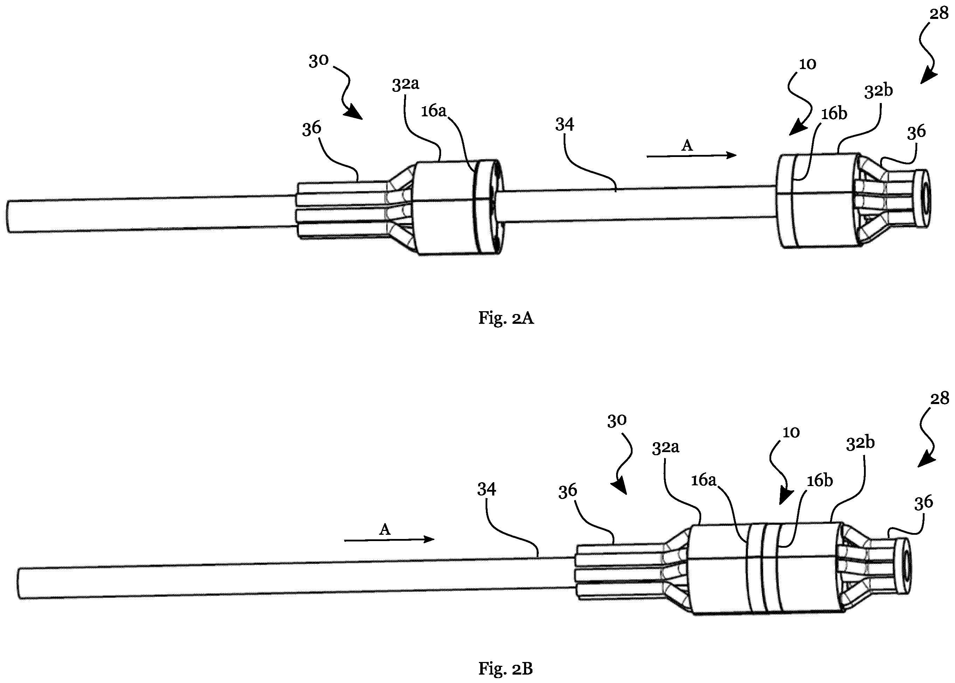

Fig. 2A, 2B schematically illustrate an example of a system 28 of an applicator 30 for transluminally assisted surgery and a corresponding implant 10 in two different configurations.

The applicator 30 comprises a first manipulator 32a and a second manipulator 32b for manipulating the first clamping element 16a and the second clamping element 16b, respectively. The first manipulator 32a and the second manipulator 32b are mounted on a common elongated applicator body 34 and are movable with respect to each other along the elongated applicator body 34. In the illustrated example, the applicator 30 comprises a plurality of suction tubes 36 running along the applicator body 34 and connected to the manipulators 32a, 32b for applying an externally supplied vacuum force at the manipulators 32a, 32b (only shown in the proximity of the first and second manipulators 32a, 32b in the figures).

An implant 10 comprising a first clamping element 16a and a second clamping element 16b is mounted on the applicator 30 via the manipulators 32a, 32b, wherein the first clamping manipulator 32a mounts the first clamping element 16a and the second manipulator 32b mounts the second clamping element 16b, the clamping elements 16a, 16b facing each other between the manipulators 32a, 32b.

The applicator body 34 may be configured as an overtube element, such as to cover an endoscope (not shown) to navigate through an anatomical lumen 14a, 140. In other words, the applicator body 34 may comprise a hollow center to be mounted on an endoscope, and the endoscope may be used to approach a site of resection in a surgical intervention. Hence, the applicator body 34 may comprise flexible segments or flexibly connected segments to adjust the shape of the overtube element to the shape of the anatomical lumen 14a, 140.

The skilled person will appreciate that the figure is merely illustrative and that additional fluid conduits, e.g. the suction tubes 36, electrical wiring (not shown) and/or force transmission cables (not shown) may run along the applicator body 34 from an aboral/proximal portion of the applicator 30 towards the manipulators 32a, 32b to transmit fluids, electric currents and/or forces through an anatomical lumen 14a, 140, such as to control a state and/or position of the manipulators 32a, 32b and/or to manipulate the clamping elements 16a, 16b. Additionally or alternatively, channels of an endoscope may be used to provide fluid connections between aboral and oral portions of the applicator 30.

In the first configuration shown in Fig. 2A, the manipulators 32a, 32b are spaced apart from each other, such that the first clamping element 16a and the second clamping element 16b are arranged facing each other for clamping disconnected tissue sections 12a, 120, e.g. of a resected bowel, between them.

The applicator 30 may be transluminally inserted, e.g. via an aboral lumen 14a and may protrude into a disconnected oral lumen 140 with the second manipulator 32b, while the first manipulator 32a may be arranged within the aboral lumen 14a. The first clamping element 16a and the second clamping element 16b may be accordingly located within the aboral lumen 14a and the oral lumen 140, respectively, in accordance with the positioning of the manipulators 32a, 32b, e.g. as previously shown in Fig. 1A.

A folded section 180 of the oral tissue section 120 can be folded over the second clamping element 16b, and the folded sections 18a, 180 may be aligned over facing surfaces 20a, 20b of the first and second clamping elements 16a, 16b. A vacuum source (not shown) may apply a vacuum to the suction tube to induce a suction force at the manipulators 32a, 32b for holding the folded tissue sections 180, 18a over the first clamping element 16a and the second clamping element 16b.

The manipulators 32a, 32b may then be moved towards each other to approach the first clamping element 16a and the second clamping element 16b towards each other, e.g. by translating the first manipulator 32a along the applicator body 34.

In the second configuration shown in Fig. 2B, the manipulators 32a, 32b are moved towards each other, such that the first clamping element 16a and the second clamping element 16b are next to each other and may clamp folded tissue sections 18a, 180 aligned over the facing surfaces 20a, 20b of the first and second clamping elements 16a, 16b, e.g. as shown in Fig. 1B.

The attractive force between the ferromagnetic portion 22 of the first clamping element 16a and the permanent magnet 24 of the second clamping element 16b, may then hold the clamping elements 16a, 16b against each other to connect the first clamping element 16a and the second clamping element 16b.

When the first clamping element 16a and the second clamping element 16b are connected to each other, a mounting of the clamping elements 16a, 16b on the manipulators 32a, 32b may be disconnected, such that the applicator 30 may be removed from the bowel. For example, the manipulators 32a, 32b may be retracted towards the elongated applicator body 34 and the applicator 30 may be retracted through the through-going passage 26 formed by the clamping elements 16a, 16b and the aboral lumen 14a.

Hence, the implant 10 may be applied and manipulated transluminally through a natural lumen 14a of the body to assist a surgeon in the creation of a surgical anastomosis with minimal surgical trauma, while the clamping elements 16a, 16b are aligned with each other for mutual engagement during deployment.

Since the clamping force of the implant 10 is based on the magnetic attraction between magnetic connection portions 22, 24 of the clamping elements 16a, 16b, the tissue sections 12a, 120 may be joined to each other without the application of comparatively large forces as opposed to stapling devices, often requiring a force of about 25 N or more to pierce the tissue of the anatomical lumen 14a, 140. In addition, the magnetic connection portions 22, 24 of the clamping elements 16a, 16b may partially self-align.

However, in the event that deployment of the implant 10 has been partially unsuccessful, e.g. when the tissue sections 12a, 120 are not joined liquid-tight, a repositioning or re-application of the clamping elements 16a, 16b of the implant 10 may require overcoming the clamping force of the implant 10 when separating the clamping elements 16a, 16b, which can be difficult with mechanical forces applied through the lumen. However, by selectively reversing a magnetization of the ferromagnetic portion 22 of the first clamping element 16a, the clamping elements 16a, 16b of the implant 10 may be alternatively separated through the local application of magnetic fields.

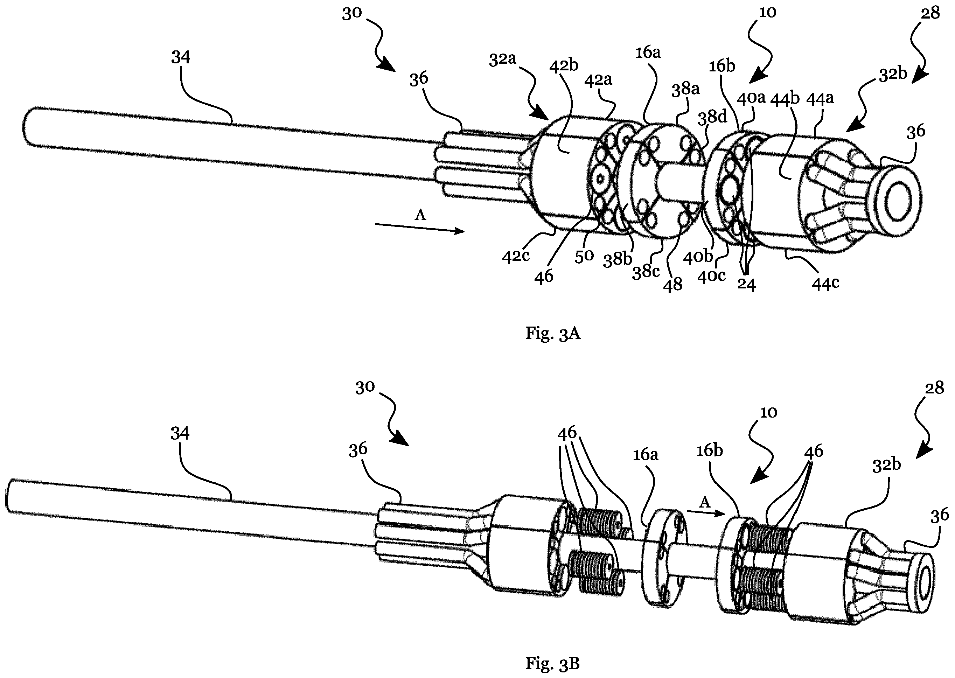

Fig. 3A and 3B show an example of an applicator 30 for mounting an implant 10 illustrated by a schematic perspective view and a partially exploded view, respectively, with the applicator 30 and the implant 10 separated from each other.

The implant 10 comprises first and second clamping elements 16a, 16b each comprising four clamping segments 38a-d, 40a-d (the fourth clamping segment 40d being occluded in the perspective views of Figs. 3A, 3B) arranged circumferentially around a common through-going passage 22 (occupied by applicator body 34). Each of the clamping segments 40a-d of the second clamping element 16b comprise a permanent magnet 24 to magnetically attract a magnetically soft ferromagnetic portion 22 (not shown) provided in each of the clamping segments 38a-d of the first clamping element 16a.

The applicator 30 comprises first and second manipulators 32a, 32b which each comprise four manipulator segments 42a-d, 44a-d (the fourth manipulator segments 42d, 44d being occluded in the perspective views of Figs. 3A, 3B) circumferentially distributed around the applicator body 34, wherein each of the manipulator segments 42a-d, 44a-d of the first and second manipulators 32a, 32b is aligned with a corresponding clamping segment 38a-d, 40a-d of the first and second clamping elements 16a, 16b, respectively.

As illustrated in Fig. 3A, the clamping segments 38a-d, 40a-d may each comprise suction conduits 46 connecting opposite axial faces of the clamping segments 38a-d, 40a-d. The suction conduits 46 may be aligned with corresponding suction ports 48 of the manipulators 32a, 32b. The suction ports 48 may in turn be connected to suction tubes 36 running along the applicator body 34 to apply a suction force at the axial faces of the clamping elements 16a, 16b.

For example, a vacuum source may apply a vacuum to one of the suction tubes 36 to induce a suction force at the axial faces 20a, 20b of the clamping elements 16a, 16b through the suction conduits 46 for holding the folded tissue sections 18a, 180 over the first clamping element 16a and the second clamping element 16b, respectively, while creating the surgical anastomosis.

Each manipulator segment 42a-d, 44a-d of the manipulators 32a, 32b comprises an associated electromagnet 50 (visibly removed from the associated manipulator segment 42a-d, 44a-d in Fig. 3B) for manipulating the first and second clamping elements 16a, 16b through selectively generated magnetic forces. In particular, the electromagnets 50 may be aligned with the magnetically soft ferromagnetic portion 22 in each of the segments 38a-d of the first clamping element 16a and configured to reverse the magnetization of the ferromagnetic portion 22 through a selectively applied magnetic field.

The electromagnet 50 may comprise coils arranged along the axial direction A of the system 28, as shown in Fig. 3B, and may comprise ferromagnetic cores (not shown) exposed towards the magnetically soft ferromagnetic portion 22 of the first clamping element 16a in order to selectively generate a magnetic field which opposes the magnetization of the permanent magnet 24.

Whether the magnetic fields generated by the electromagnet 50 reverses the magnetization of the ferromagnetic portion 22 of the first clamping element 16a may depend on a coupling strength between the electromagnet 50 and the ferromagnetic portion 22 as well as the current profile applied to the electromagnet 50.

The coupling strength between the electromagnet 50 and the ferromagnetic portion 22 may depend on the geometric configuration of the first manipulator 32a and the first clamping element 16a. Preferably, the ferromagnetic core of the electromagnet 50 is exposed at the periphery of the first manipulator 32a, such as to lie next to the ferromagnetic portion 22 of the first clamping element 16a. For example, the ferromagnetic core of the electromagnet 50 may be arranged such that it contacts the first clamping element 16a, when the first clamping element 16a is mounted on the first manipulator 32a.

In some embodiments, the first clamping element 16a and the first manipulator 32a are configured to interlock in a mounting configuration, such that a relative motion between the first clamping element 16a and the first manipulator 32a along the circumferential direction with respect to the axial direction A is prevented. For example, the first manipulator 32a may comprise guiding rails (not shown) for mounting the first clamping element 16a, such as to constrain a relative motion of the first clamping element 16a and the first manipulator 32a to the axial direction A and to prevent a movement of the first clamping element 16a along the circumferential direction.

In some embodiments, the first manipulator 32a comprises a recess aligned with the coils of the electromagnet 50, such that a ferromagnetic portion 22 of the first clamping element 16a may protrude into the coils of an electromagnet 50 of the first manipulator 32a to prevent a circumferential motion of the ferromagnetic portion 22 of the first clamping element 16a with respect to the electromagnet 50 and to increase a coupling between the electromagnet 50 and the ferromagnetic portion 22. Additionally or alternatively, the ferromagnetic core of an electromagnet 50 may protrude out of the coils of the electromagnet 50, such that the ferromagnetic core may protrude into the ferromagnetic portion 22 of the first clamping element 16a.

In some embodiments, the first manipulator 32a comprises a mechanical locking mechanism (not shown) configured to selectively prevent a relative movement between the first clamping element 16a and the first manipulator 32a along the axial direction A. For example, the first manipulator 32a may comprise a retractable holding bracket (not shown) configured to hold the first clamping element 16a and the first manipulator 32a to each other in a first configuration and to release the first clamping element 16a in a second configuration. Accordingly, the mechanical locking mechanism may be selectively activated to prevent the separation of the first clamping element 16a and the first manipulator 32a while a magnetic field generated by the electromagnet 50 is building up to reverse the magnetization of the ferromagnetic portion 22 of the first clamping element 16a.

As another example, a locking mechanism may selectively prevent relative movement between the first and second manipulators 32a, 32b along the elongated applicator body 34, such as to prevent the first clamping element 16a from being separated from the first manipulator 32a while the magnetization of the ferromagnetic portion 22 is reversed.

In addition, the current profile applied to the electromagnet 50 may be adapted to selectively induce a separation of the first clamping element 16a and the second clamping element 16b. For example, the current profile may comprise currents of opposite polarity to initially attract the ferromagnetic portion 22, while it is magnetically coupled to the magnetically hard connection portion 24 of the second clamping element 16b, and to subsequently reverse the magnetization of the ferromagnetic portion 22 of the first clamping element 16a.

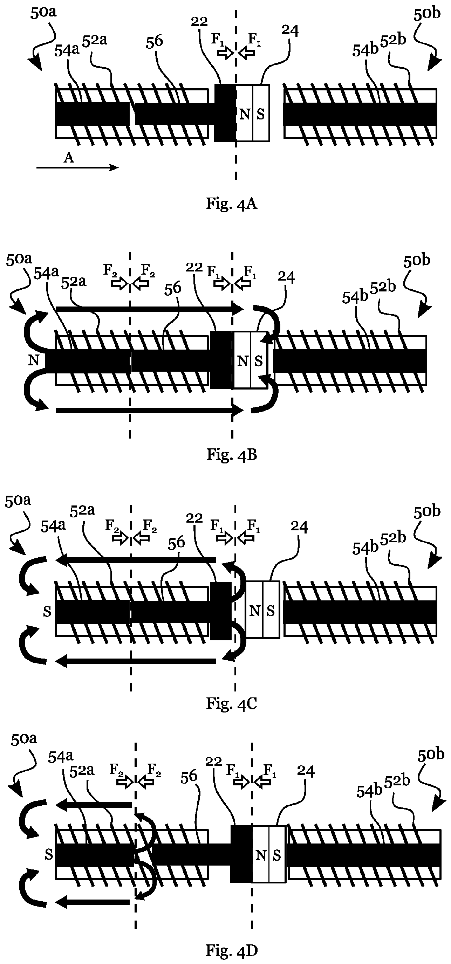

Figs. 4A-D illustrate different stages in an interaction between facing electromagnets 50a, 50b, a ferromagnetic portion 22 and a permanent magnet 24 in different exemplary configurations.

A first electromagnet 50a comprises a coil 52a and a ferromagnetic core 54a, wherein the ferromagnetic core 54a is recessed into the coil 52a, such that the coil 52a partially circumscribes a recessed opening for receiving a protruding portion 56 of the ferromagnet 22. The first electromagnet 50a may correspond to an electromagnet 50 of the first manipulator 32a in the previously described examples for manipulating a first clamping element 16a and the ferromagnet 22 may correspond to the ferromagnetic portion 22 of the first clamping element 16a.

A second electromagnet 50b equally comprises a coil 52b and a ferromagnetic core 54b, and faces the first electromagnet 50a. The second electromagnet 50b is arranged adjacent to the permanent magnet 24 and may correspond to an electromagnet 50 of the second manipulator 32b in the previously described examples for manipulating the permanent magnet 24 of the second clamping element 16b. The second electromagnet 50b may be driven with oppositely polarized currents to selectively attract or repel the permanent magnet 24.

In Fig. 4A, no current is applied to the electromagnets 50a, 50b, and the ferromagnet 22 and the permanent magnet 24 lie next to each other. The permanent magnet 24 induces a magnetization in the ferromagnet 22 which is aligned with the magnetization of the permanent magnet 24 (indicated by the magnetic poles "N"/ "S") resulting in a mutual magnetic attraction with a force F₁. The skilled person will appreciate that the permanent magnet 24 may also partially magnetically polarize the ferromagnetic cores 54a, 54b of the first and second electromagnet 50a, 50b, such that the electromagnets 50a, 50b, the ferromagnet 22 and the permanent magnet 24 may be joined to each other, e.g. in the absence of a current in the electromagnets 50a, 50b or of external forces.

In Fig. 4B a first current is applied to the coil 52a of the first electromagnet 50a to generate a magnetic field (indicated with solid black arrows, magnetic pole of the first electromagnet 50a indicated with "N" on the left side) with the coil 52a which is aligned with the magnetization of the permanent magnet 24, such that the ferromagnet 22 and the permanent magnet 24 are attracted towards the first electromagnet 50a, in particular to the ferromagnetic core 54a of the first electromagnet 50a.

The protruding portion 56 of the ferromagnet 22 may be pulled into the coil 52a of the first electromagnet 50a and towards the ferromagnetic core 54a of the first electromagnet 50a, thereby reducing a distance between the ferromagnetic core 54a and the ferromagnet 22. In addition, the first electromagnet 50a may increase the magnetization of the ferromagnetic core 54a and the ferromagnet 22. As a result, the ferromagnetic core 54a and the ferromagnet 22 may be joined to each other with an attractive magnetic force F₂.

In Fig. 4C, a second current is applied to the coil 52a of the first electromagnet 50a to generate a magnetic field (indicated with solid black arrows, magnetic pole of the first electromagnet 50a indicated with "S" on the left side) with the coil 52a which is aligned with, but opposite to the magnetization of the permanent magnet 24 following an application of the first current.

Accordingly, the magnetic field generated by the coil 52a of the electromagnet 50a may exert a reverting effect on the magnetization of the ferromagnetic core 54a and the ferromagnet 22, in particular on the protruding portion 56 of the ferromagnet 22. In addition, the magnetic field generated by the coil 52a of the electromagnet 50a may repel the permanent magnet 24 as well as the ferromagnet 22 prior to a reversal of the magnetization in the ferromagnet 22.

The magnetic field in the first electromagnet 50a may build up progressively, e.g. due to a self-inductance of the first electromagnet 50a, such that the magnetization of the ferromagnetic core 54a and the ferromagnet 22 may also be progressively reversed in response to the applied second current, e.g. in accordance with a hysteresis of the ferromagnetic core 54a and the ferromagnet 22.

During the reversal of the magnetization of the ferromagnetic core 54a and the ferromagnet 22, the attractive magnetic force F₂ between the ferromagnetic core 54a and the ferromagnet 22 may decrease towards zero, when the magnetization of the ferromagnetic core 54a is close to zero. When the magnetization of the ferromagnetic core 54a and the ferromagnet 22 is reversed, the attractive magnetic force F₂ between the ferromagnetic core 54a and the ferromagnet 22 may increase again.

At the same time, the attractive magnetic forces F₁ between the ferromagnet 22 and the permanent magnet 24 may also decrease while the magnetization of portions of the ferromagnet 22 may be reversed and may counteract the attractive magnetic forces F₁ induced by the permanent magnet 24.

Depending on the magnetic coupling strength between the first electromagnet 50a and the ferromagnet 22, the ferromagnet 22 may then either be expelled from the first electromagnet 50a, or as shown in Fig. 4C, the magnetization of the ferromagnet 22 may be reversed and the permanent magnet 24 may be repelled by the ferromagnet 22.

In Fig. 4D, the second current is applied to the coil 52a of the first electromagnet 50a to generate a magnetic field which is aligned with but opposite to the magnetization of the permanent magnet 24, but without a previous application of the first current.

In the case where the first current was not applied previously, before the second current is applied, the magnetization of the ferromagnetic core 54a and the ferromagnet 22 may be smaller than in the configuration depicted in Fig. 4B, such that the magnetic attractive force F₂ between them may be smaller or close to zero. When the second current is applied in this configuration, a repellant force on the ferromagnet 22 may be generated by the coil 52a, such that the ferromagnet 22 may be displaced away from the ferromagnetic core 54a and out of the coil 52a.

Accordingly, based on the employed current profile, the clamping elements 16a, 16b may be selectively separated from each other as illustrated in Fig. 4C or selectively disconnected from the first manipulator 32a as illustrated in Fig. 4D.

The Inventors observed that the dynamics of the separation/repulsion of the ferromagnet 22 and the permanent magnet 24 could be reliably reproduced in their experiments in an arrangement similar to the one shown in Figs. 4A-4D.

The permanent magnet 24 was implemented with a disk-shaped NdFeB based permanent magnet with a diameter of 1 cm and a thickness of about 3 mm, and the ferromagnet 22 was implemented with an integral iron piece with a low carbon content featuring a disk-shaped head facing the permanent magnet 24 with a diameter of about 1 cm and a cylindrical bolt with a diameter of about 5 mm forming a protruding portion 56 protruding into an electromagnet 50a by about 1 cm as shown in Fig. 4A. The electromagnet 50a comprised a coil 52a with a length of about 2 cm, a diameter of about 1,2 cm and about 300 windings of a copper wire, and a ferromagnetic core 54a occupying one half of the longitudinal extension of the coil 52a, such that the bolt of the ferromagnet 22 and the ferromagnetic core 54a meet substantially at the center of the coil 52a in the configuration of Figs. 2A, B.

The inventors observed a reliable separation of the ferromagnet 22 and the permanent magnet 24 by applying a first current as in Fig. 4A with a current source applying a voltage of about 15 Volts and a current of about 3A and subsequently reversing the polarity of the applied voltage with a switch to apply a second current to the coils 52a of the electromagnet 50a as in Fig. 4A, while at the same time applying a low mechanical force to separate the electromagnets 50a, 50b from each other. The separation results in the ferromagnet 22 adhering to the first electromagnet 50a while the permanent magnet 24 adheres to the ferromagnetic core 54b of the second electromagnet 50b as shown in Fig. 4C.

Conversely, by applying the second current to the first electromagnet 50a without prior application of the first current, the ferromagnet 22 could be reliably expelled from the first electromagnet 50a, resulting in the ferromagnet 22 adhering to the permanent magnet 24 which in turn adhered to the ferromagnetic core 54b of the second electromagnet 50b as shown in Fig. 4D.

When the implant 10 is in the configuration of Fig. 4D, the ferromagnet 22 and the permanent magnet 24 may be separated from the second electromagnet 50b by generating a magnetic field with the coil(s) 52b of the second electromagnet 50b which is opposite to the magnetization of the permanent magnet 24. Hence, the clamping elements 16a, 16b of the implant may be selectively separated or disconnected from the applicator 30 with magnetic forces.

While the preliminary experiments of the inventors focused on a ferromagnet 22 protruding into the coil 52a of the first electromagnet 50a, the skilled person will appreciate that the magnetic coupling between the first electromagnet 50a and the magnetically soft ferromagnetic portion 22 of a first clamping element 16a and between the ferromagnetic portion 22 and a permanent magnet 24 of a second clamping element 16b may be adjusted through various parameters and may not require the magnetically soft protruding portion 56 protruding into the coils 52a of the first electromagnet 50a by more than 30% of the length of the coils 52a, by more than 10% of the length of the coils 52a, or at all. For example, a respective magnetic coupling may be adjusted based on a thickness of a (bio-compatible) coating layer covering the permanent magnet 24, the ferromagnetic portion 22 and/or the ferromagnetic core 54a of the electromagnet 50a, or the shape of the permanent magnet 24 and the ferromagnetic portion 22.

Further, the skilled person will appreciate that, due to a progressively decaying magnetization of the material of the ferromagnetic portion 22 and/or the ferromagnetic core 54a of the electromagnet 50a, the second current in the coil 52a of the first electromagnet 50a may not need to be applied directly after the first current, but may also be applied within a separation time frame after the application of the first current while still inducing a separation of the permanent magnet 24 and the ferromagnetic portion 22.

After the final deployment of the implant 10, the applicator 30 comprising the electromagnets 50a, 50b may be retracted through the common central passage 26 of the clamping elements 16a, 16b of the implant 10. One or both of the implant 10 and the applicator 30 may be convertible between different configurations featuring different perimeters, such as to enable retracting the applicator 30 through the through-going passage 26. In some embodiments, the perimeter of the clamping elements 16a, 16b may be radially expanded to increase a diameter of the central through-going passage 26. In some embodiments, the first manipulator 32a and/or the second manipulator 32b may be convertible between different configurations featuring a different outside perimeter with respect to the elongated applicator body 34, such as to enable retracting the applicator 30 through the central passage 26 of the implant 10 in a collapsed configuration following deployment.

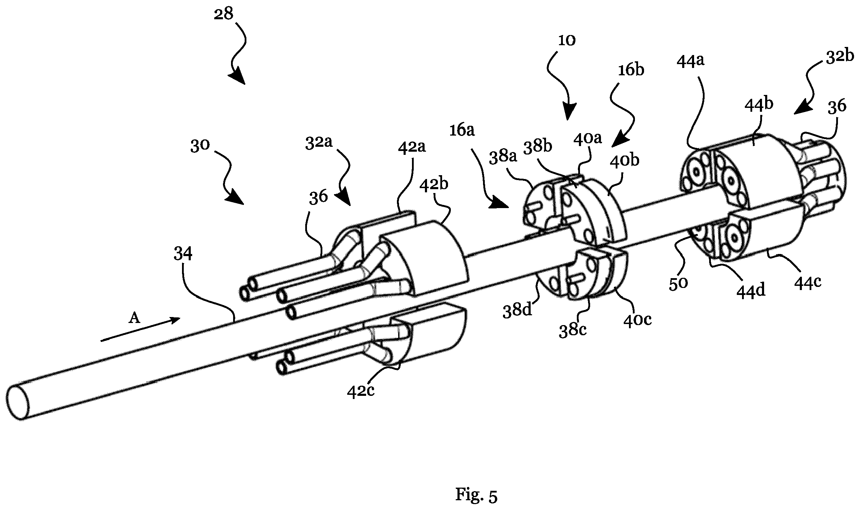

Fig. 5 schematically illustrates a system 28 of an applicator 30 and an implant 10, wherein each of the applicator 30 and the implant 10 may be convertible between different configurations to adjust a perimeter of the applicator 30 and the implant 10, respectively.

Similar to the example shown in Fig. 3A, the implant 10 comprises first and second clamping elements 16a, 16b each comprising four clamping segments 38a-d, 40a-d (the fourth clamping segment 40d being occluded in the perspective view of Fig. 5) arranged circumferentially around a common through-going passage 26 (not explicitly shown). Each of the clamping segments 40a-d of the second clamping element 16b comprises a permanent magnet 24 (not shown) to magnetically attract a magnetically soft ferromagnetic portion 22 (not shown) provided in each of the clamping segments 38a-d of the first clamping element 16a.

The applicator 30 comprises first and second manipulators 32a, 32b each comprising four manipulator segments 42a-d, 44a-d (the fourth manipulator segment 42d being occluded in the perspective view of Fig. 5) circumferentially distributed around the applicator body 34, wherein each of the manipulator segments 42a-d, 44a-d of the first and second manipulators 32a, 32b are aligned with a corresponding clamping segment 38a-d, 40a-d of the first and second clamping elements 16a, 16b, respectively. The manipulator segments 42a-d, 44a-d of the first and second manipulators 32a, 32b are configured to mount a respective clamping segment 38a-d, 40a-d of the first and second clamping elements 16a, 16b, and each comprises an electromagnet 50 for manipulating the clamping elements 16a, 16b through selectively applied magnetic forces.

The clamping segments 38a-d, 40a-d of the first and second clamping elements 16a, 16b are illustrated in a partially expanded configuration, wherein the clamping segments 38a-d, 40a-d are spaced from each other along a circumferential direction and are radially spaced from the central applicator body 34. The clamping segments 38a-d, 40a-d may be held in a given relative geometric arrangement by adjustable joining segments (not shown) connecting adjacent clamping segments 38a-d, 40a-d to each other, such as to form a closed loop around a central through-going passage 26. For example, the clamping segments 38a-d, 40a-d of the first and second clamping elements 16a, 16b may be connected via convertible cavities (not shown) which can be selectively filled with a filling medium to adjust a spacing between adjacent clamping segments 38a-d, 40a-d. Additionally or alternatively, the clamping segments 38a-d, 40a-d may be connected by a mechanical locking mechanism (not shown) to selective lock the relative arrangement of the clamping segments 38a-d, 40a-d at a desired spacing.

Similarly, the applicator segments 42a-d of the first manipulator 32a are shown in an expanded configuration, in which the applicator segments 42a-d are radially spaced from the elongated applicator body 34 extending along the axial direction A. Conversely, the applicator segments 44a-d of the second manipulator 32b are in a substantially closed configuration adjacent to the applicator body 34.

The applicator segments 42a-d, 44a-d of the first and second manipulators 32a, 32b may each be selectively expandable to adjust a spacing between the applicator segments 42a-d, 44a-d and the applicator body 34 along the radial direction. For example, each of the applicator segments 42a-d, 44a-d may be mounted on a pivotable manipulator arm (not shown), which may be selectively pivotable with respect to the applicator body 34 to adjust a spacing between the applicator segments 42a-d, 44a-d and the applicator body 34.

For the creation of a surgical anastomosis with the implant 10, an operator may transluminally insert the applicator 30 into the anatomical lumen 12a, 120 with each of the first and second manipulators 32a, 32b holding the first and second clamping elements 16a, 16b of the implant 10, respectively, in a closed configuration. At the site of the resection, the operator may operate a first drive mechanism (not shown), such as a cable pull or hydraulic mechanism, of the applicator 30 to space the applicator segments 42a-d, 44a-d away from applicator body 34, thereby expanding the clamping elements 16a, 16b towards a deployment state by spacing neighboring clamping segments 38a-d, 40a-d apart from each other. Tissue sections 18a, 180 may be folded over the clamping elements 16a, 16b and held in place with a vacuum applied to suction tubes 36 of the applicator 30.

By driving a second driving mechanism (not shown), the first and second manipulators 32a, 32b may be selectively approached towards each other to engage the clamping elements 16a, 16b to each other via the magnetic attraction of the permanent magnets 24 of the second clamping element 32b onto the ferromagnetic portions 22 of the first clamping element 32a.

The first and second manipulators 32a, 32b may be subsequently disconnected from the clamping elements 16a, 16b by selectively applying currents to the electromagnets 50 to arrive at the configuration depicted in Fig. 5. In the following, at least one of the first and second manipulators 32a, 32b may be retracted towards the applicator body 34 to retract the applicator 34 through the central through-going passage 26 of the implant 10.

It is noted that the foregoing description was discussed for the purpose of illustration and should not be construed as limiting. The skilled person will in particular appreciate that the first and second manipulators 32a, 32b and the clamping elements 16a, 16b may be expanded towards a deployment state before or after engaging the ferromagnetic portions 22 of the first clamping element 32a and the permanent magnets 24 of the second clamping element 16b. Further, the first and second manipulators 32a, 32b are illustrated with an example of manipulators 32a, 32b each having four manipulator segments 42a-d, 44a-d for mounting corresponding clamping elements 16a, 16b each having a corresponding number of four clamping segments 38a-d, 40a-d. However, the skilled person will appreciate that in some embodiments, the manipulators 32a, 32b comprise less than four manipulator segments 42a-d, 44a-d, such as two or three manipulator segments 42a-d, 44a-d, or more than four manipulator segments 42a-d, 44a-d, e.g. five, six, or eight manipulator segments 42a-d, 44a-d, such as to mount and manipulate clamping elements 16a, 16b having a corresponding number of clamping segments 38a-d, 40a-d and/or having a corresponding number of magnetically soft/hard connection portions 22, 24. In addition, the skilled person will appreciate that the magnetic moments of the permanent magnet, and the magnetic field generated by the electromagnets have been described to be aligned along the direction A to describe one embodiment of the invention. However, the magnetization of the permanent magnets 24 may also be transverse to the axial direction A of the applicator 30 and the electromagnet(s) and/or their ferromagnetic cores 54a, 54b may accordingly be aligned to generate a magnetic field transverse to the axial direction A of the applicator 30.

The description of the preferred embodiments and the figures merely serve to illustrate the invention and the beneficial effects associated therewith, but should not be understood to imply any limitation. The scope of the invention is to be determined solely by the appended claims.

### LIST OF REFERENCE SIGNS

- 10: implant
- 12a, 120: disconnected aboral and oral bowel sections
- 14a, 140: aboral and oral lumen
- 16: clamping element
- 16a, 16b: first and second clamping element
- 18a, 180: folded tissue sections
- 20a, 20b: axial face/facing surface of the first and second clamping element
- 22: ferromagnet/ferromagnetic portion
- 24: permanent magnet
- 26: through-going passage
- 28: system
- 30: applicator
- 32a, 32b: first and second manipulator
- 34: elongated applicator body
- 36: suction tubes
- 38a-d: clamping segments of the first clamping element
- 40a-d: clamping segments of the second clamping element
- 42a-d: applicator segments of the first manipulator
- 44a-d: applicator segments of the second manipulator
- 46: suction conduits
- 48: suction ports
- 50, 50a, 50b: electromagnet
- 52: coils
- 54: ferromagnetic core
- 56: protruding portion

## Claims

1. A clamping device (10) for joining flexible tubular walls (12a, 120), the clamping device (10) comprising a first clamping element (16a) and a second clamping element (16b), wherein the first clamping element (16a) comprises a magnetically soft connection portion (22),
wherein the second clamping element (16b) comprises a magnetically hard connection portion (24), and
wherein the first clamping element (16a) and the second clamping element (16b) are configured to define a common central through-going passage (26) through the first clamping element (16a) and the second clamping element (16b), when the magnetically soft connection portion (22) of the first clamping element (16a) is coupled to the magnetically hard connection portion (24) of the second clamping element (16b).

2. The clamping device (10) according to claim 1, wherein the clamping device (10) is configured as a transluminally applied implant for joining anatomical lumina (14a, 140), and wherein the first clamping element (16a) and the second clamping element (16b) in particular each comprise a biocompatible coating.

3. The clamping device (10) according to claim 1 or 2, wherein the magnetic coercitivity of the magnetically soft connection portion (22) is smaller than 1 kA/m, and/or
wherein the magnetic coercitivity of the magnetically hard connection portion (24) is greater than 1 kA/m, and/or
wherein a relative magnetic permeability of the magnetically soft connection portion (22) is larger than 50, in particular larger than 300, preferably larger than 1000.

4. The clamping device (10) according to any one of the preceding claims, wherein the first clamping element (16a) comprises a plurality of magnetically soft connection portions (22) and the second clamping comprises a plurality of magnetically hard connection portions (24), wherein the plurality of magnetically soft connection portions (22) and the magnetically hard connection portions (24) are arranged circumferentially around the common through-going passage (26) of the first clamping element (16a) and the second clamping element (16b), respectively, to magnetically couple the first clamping element (16a) and the second clamping element (16b).

5. The clamping device (10) according to any one of the preceding claims, wherein the first clamping element (16a) comprises a magnetic coupling section for magnetically coupling to the magnetically soft connection portion (22) at a coupling side, wherein the coupling side is different from a joining side (20a) for coupling the first clamping element (16a) and the second clamping element (16b), in particular opposite the joining side (20a),
wherein the first clamping element (16a) is preferably configured such that a magnetic coupling of the magnetically soft connection portion (22) to an external magnet (50, 50a) at the coupling section is equal to or greater than the magnetic coupling of the magnetically soft connection portion (22) to an external magnet at the joining side (20a),
wherein the magnetic coupling section in particular comprises a protruding portion (56) of the magnetically soft connection portion (22), wherein the protruding portion (56) protrudes away from the joining side (20a) of the first clamping element (16a), and/or
wherein the magnetic coupling section in particular comprises a recess protruding towards, and preferably into, the magnetically soft connection portion (22) for receiving a magnetic coupling portion of an applicator (30).

6. The clamping device (10) according to any one of the preceding claims, wherein the first clamping element (16a) and/or the second clamping element (16b) comprise(s) internal suction conduits (46) to apply a suction force at a joining side (20a) of the first clamping element (16a) and/or the second clamping element (16b) for holding an end portion of one of the flexible tubular walls (12a, 120) to the joining side (20a).

7. The clamping device (10) according to any one of the preceding claims, wherein the first clamping element (16a) and the second clamping element (16b) are each convertible between a first and a second configuration to change a perimeter of the common through-going passage (26).

8. An applicator (30) for manipulating a clamping device (10) with a first clamping element (16a) and a second clamping element (16b), the first clamping element (16a) and the second clamping element (16b) comprising a magnetically soft connection portion (22) and a magnetically hard connection portion (24), respectively, wherein the applicator (30) comprises:
an applicator body (34),
a first manipulator (32a) and a second manipulator (32b) mounted on the applicator body (34) and configured to be displaced with respect to each other along the applicator body (34),
wherein the first manipulator (32a) and the second manipulator (32b) are configured to hold the first clamping element (16a) and the second clamping element (16b), respectively, in an arrangement in which the first clamping element (16a) and the second clamping element (16b) are facing each other,
wherein the first manipulator (32a) comprises an electromagnet (50, 50a) for coupling to the magnetically soft connection portion (22) of the first clamping element (16a), and a control system configured to selectively apply a separation current profile to the electromagnet (50, 50a) of the first manipulator (32a) to reverse a magnetization of the magnetically soft connection portion (22) of the first clamping element (16a) while the first clamping element (16a) is magnetically coupled to the second clamping element (16b), such that the first clamping element (16a) and the second clamping element (16b) are separated from each other through magnetic forces.

9. The applicator (30) of claim 8, wherein the first manipulator (32a) and the second manipulator (32b) each comprise an electromagnet (50, 50a, 50b) for coupling to the magnetically soft connection portion (22) of the first clamping element (16a) and to the magnetically hard connection portion (24) of the second clamping element (16b), respectively.

10. The applicator (30) of claim 8 or 9, wherein the electromagnet (50, 50a) of the first manipulator (32a) comprises a magnetic core (54a) exposed towards the magnetically soft connection portion (22) of the first clamping element (16a) and is configured to magnetically couple to the magnetically soft connection portion (22) of the first clamping element (16a), and/or
wherein the first manipulator (32a) comprises a recess protruding towards, and in particular into, the electromagnet (50, 50a) for receiving a protruding coupling portion of the first clamping element, and/or
wherein the first manipulator (32a) comprises a protruding portion magnetically coupled to the electromagnet (50, 50a), wherein the protruding portion protrudes from the electromagnet (50, 50a) towards the second manipulator (32b).

11. The applicator (30) of any one of claims 8 to 10, wherein the control system is configured to selectively apply a first current having a first polarity to the electromagnet (50, 50a) of the first manipulator (32a) in a first time slot and to selectively apply a second current having an opposite second polarity to the electromagnet (50, 50a) of the first manipulator (32a) in a second time slot to separate the first clamping element (16a) and the second clamping element (16b), and/or
wherein the first manipulator (32a) comprises a holding bracket to selectively retain the first clamping element (16a) along an axial direction of the applicator (30).

12. The applicator of any one of claims 8 to 11, wherein the applicator body (34) comprises an accommodating passage to mount the implant applicator (30) on the outer face of an endoscope, and/or
wherein the applicator body (34) comprises flexible or flexibly connected segments to navigate through an anatomical lumen (14a, 140), and/or
wherein the outer diameter of the implant applicator (30) is smaller than 60 mm, in particular smaller than 40 mm, preferably smaller than 30 mm, in a navigation configuration of the implant applicator (30) to navigate through an anatomical lumen (14a, 140), and/or
wherein the applicator (30) comprises an imaging device configured to capture an image at or close to the first manipulator (32a) and/or the second manipulator (32b).

13. The applicator of any one of claims 8 to 12, further comprising a drive mechanism to move the first manipulator (32a) and/or the second manipulator (32b) with respect to the applicator body (34), wherein the drive mechanism in particular comprises a fluid conduit for transmitting a hydraulic or pneumatic force to the first manipulator (32a) and/or the second manipulator (32b), and/or
a cable pull to transmit a mechanical force to the first manipulator (32a) and/or the second manipulator (32b), and/or
wherein the first manipulator (32a) and/or the second manipulator (32b) are convertible between a first configuration and a second configuration to deploy the clamping device (10) in the first configuration and to retract the applicator (30) through a central through-going passage (26) of the clamping device (10) in the second configuration, and/or
wherein the applicator (30) further comprises suction conduits (36) to apply a suction force at the first manipulator (32a) and/or the second manipulator (32b).

14. A system comprising a clamping device (10) and an applicator (30) for manipulating the clamping device (10), the clamping device (10) comprising a first clamping element (16a) and a second clamping element (16b) comprising a magnetically soft connection portion (22) and a magnetically hard connection portion (24), respectively, wherein the applicator (30) comprises:
an applicator body (34),
a first manipulator (32a) and a second manipulator (32b) mounted on the applicator body (34) and configured to be displaced with respect to each other along the applicator body (34),
wherein the first manipulator (32a) and the second manipulator (32b) are configured to hold the first clamping element (16a) and the second clamping element (16b), respectively,
wherein the first manipulator (32a) comprises an electromagnet (50, 50a) for coupling to the magnetically soft connection portion (22) of the first clamping element (16a).

15. A method of driving an applicator (30) for a clamping device (10) with a pair of separable first and second clamping elements (16a, 16b) having magnetically soft and magnetically hard connection portions (22, 24), respectively, the method comprising the steps of:
applying a current having a first current polarity at a first electromagnet (50, 50a) of the applicator (30) while the first clamping element (16a) is held at the first electromagnet (50, 50a), and
applying a current having an opposite second current polarity at the first electromagnet (50, 50a) of the applicator (30) for separating the first clamping element (16a) and the second clamping element (16b).
